# FASCICULE DE BREVET EUROPEEN

(11) **EP 0 910 994 B1**
(45) Date de publication et mention de la délivrance du brevet: **23.07.2003**
(21) Numéro de dépôt: 98402508.0
(22) Date de dépôt: 09.10.1998
(51) Int. Cl.: A61B 19/08, A61F 13/44, A61F 13/15

(54) **Procédé de fabrication en continu de compresses ou champs opératoires à usage unique et compresse ou champ opératoire obtenu par ce procédé**
Verfahren zur kontinuierlichen Herstellung einer Kompresse und eines Chirurgischen Abdecktuchs für einmalige Verwendung und so hergestelltes Erzeugnis
Method for continuously producing single use surgical drape or compress, and product therefrom

(30) Priorité: 17.10.1997 FR 9713018
(43) Date de publication de la demande: 28.04.1999
(73) Titulaire: Laboratoire Hydrex (SA), 69550 Amplepuis (FR)
(72) Inventeur: Bonnassieux, Gilles, 69290 Pollionnay (FR)
(74) Mandataire: Lachat, Salomé

(56) Documents cités:
- EP-A- 0 267 861
- EP-A- 0 702 938
- GB-A- 716 923
- US-A- 4 157 719

## Description

La présente invention est relative à un procédé de fabrication en continu de compresses ou champs opératoires à usage unique, constitués de plusieurs couches superposées alternativement en gaze de coton hydrophile et en non tissé hydrophile, les couches extérieures étant en gaze de coton hydrophile, et, à la compresse ou au champ opératoire obtenu par ce procédé.

Des champs opératoires à usage unique de ce type sont utilisés notamment en milieu hospitalier à des fins chirurgicales, par exemple comme champ de paroi recouvrant les bords de la plaie après l'incision et avant la pose des écarteurs. Ils peuvent être utilisés également comme champs abdominaux pour recliner les viscères ou les anses intestinales. Lorsque humidifiés préalablement, on peut les utiliser pour protéger un organe fragile du dessèchement.

On connaît des champs opératoires en coton, en polyester/coton ou en polyester/microfibre, qui sont relavés et désinfectés après chaque usage, repliés, reconditionnés et restérilisés, d'où un risque de contamination accidentelle dans les blanchisseries lors des manipulations des champs opératoires maculés. Ces champs opératoires présentent encore l'inconvénient de donner naissance à des particules formées de bourres de coton susceptibles de provoquer des infections.

Pour pallier ces inconvénients inhérents aux champs opératoires connus à usages répétés après reconditionnement, le brevet français 2724838 et la demande de brevet européen 0702938 A1 appartenant à la demanderesse, proposent un champ opératoire à usage unique constitué de plusieurs nappes superposées alternativement en gaze de coton hydrophile et en voile non tissé. Ce champ opératoire est fabriqué à l'aide d'un procédé se distinguant essentiellement par les étapes suivantes : sur une nappe unique continue de gaze de coton hydrophile sont déposées deux nappes de non tissé dont la largeur de chacune correspond à un tiers de la nappe de gaze de coton hydrophile de manière à laisser libre un tiers de la largeur de cette dernière; la bande multicouche est obtenue par pliage de manière à ce que la gaze de coton hydrophile forme les couches extérieures de la bande multicouche.

Contrairement aux produits connus présentant une certaine similarité, ce champ opératoire qui est fabriqué sans utilisation d'adhésif pour l'assemblage des différentes couches, présente l'avantage d'un contact sur la plaie avec une fibre naturelle qui ne «peluche» pas et de la capacité d'absorption d'une fibre non tissée constituée par exemple d'un composé de viscose absorbante et de polypropylène, d'un composé de polyester et de viscose, d'un polyester ou d'un polyamide.

En effet, l'association de matériaux différents, savoir un matériau tissé et un matériau non tissé, permet de cumuler les avantages respectifs des deux : le matériau tissé présente une résistance identique à l'état sec et à l'état mouillé, alors que le matériau non tissé, mouillé, a une résistance plus faible. En outre, la gaze de coton hydrophile assure un « toucher tissu » et sa grande résistance à la tension confère au produit fini sa solidité. Par contre, le matériau non tissé présente une très grande capacité d'absorption. Des champs opératoires de ce type possèdent un pouvoir d'absorption deux fois plus grand qu'un champ opératoire classique en matériau tissé tout en présentant une très grande résistance.

Ce type de produit développé par la demanderesse donne entière satisfaction à l'usage. Cependant, pour tenir compte des impératifs économiques, il est souhaitable de s'orienter vers un procédé de fabrication beaucoup plus simple que celui proposé par la demanderesse dans les titres de propriété industrielle cités plus haut, afin de réduire le coût de fabrication du produit et de le rendre plus compétitif.

Par suite, la présente invention a pour objet un procédé de fabrication en continu de compresses ou champs opératoires du type précédemment décrit, associant deux matériaux différents, à savoir de la gaze de coton hydrophile et du non tissé, qui soit plus simple à mettre en oeuvre et permette de réduire le coût de fabrication du produit.

Pour atteindre ces objectifs, la présente invention propose un procédé de fabrication en continu qui se distingue par les étapes suivantes:
- confection d'une bande ou nappe multicouche unique continue par déroulement simultané de bandes ou nappes de gaze de coton hydrophile et de voile de non tissé à partir de dérouleurs auxquels peut être associé au moins un dispositif de réglage automatique de la tension des bandes, lesdites bandes passant ensemble par un dispositif d'entraînement motorisé avant d'atteindre les stations de confection des champs opératoires individuels,
- pose, par déroulement à partir de dérouleurs, de rubans ou biais continus en non tissé sur les bords longitudinaux de la bande multicouche, lesdits biais étant repliés autour des bords longitudinaux de la bande multicouche sur les nappes extérieures pour former ourlet,
- assemblage, à une station de soudage, des rubans ou biais repliés autour des bords longitudinaux de la bande multicouche par des soudures par ultrasons traversant les branches des biais repliés et toutes les couches de la bande multicouche,
- découpe transversale à longueur des champs opératoires individuels à une station de découpe et éventuellement aspiration des poussières par un dispositif d'aspiration,
- reprise en perpendiculaire du champ opératoire découpé par un dispositif d'entraînement,
- pose de rubans ou biais formant ourlet autour des bords transversaux du champs opératoire découpé, lesdits rubans ou biais repliés étant solidarisés par des soudures par ultrasons.

Si les éléments constitutifs essentiels de ce champ opératoire obtenus conformément à l'invention sont semblables à ceux du champ opératoire obtenu par le procédé décrit dans la demande de brevet EP 0 702 938 appartenant à la demanderesse, il convient de remarquer que le procédé de fabrication antérieur décrit dans la demande de brevet EP 0 702 938 diffère totalement de celui, objet de la présente invention. Conformément au procédé de la demande de brevet EP 0 702 938 le champ opératoire est fabriqué par pliage à partir d'une nappe de gaze hydrophile unique sur laquelle ont été posées une ou deux nappes de non tissé hydrophile. Au contraire, le procédé conforme à la présente invention n'a recours à aucun pliage. Par conséquent, en raison même du procédé de fabrication se distinguant par un pliage d'une nappe unique, certains bords des couches superposées sont des pliures et non des bords francs comme pour le champ opératoire fabriqué selon la présente invention.

Selon un mode de réalisation préférentiel de l'invention, l'assemblage des nappes composant la bande multicouche et la fixation des rubans ou biais repliés autour des quatre côtés des champs opératoires pour former ourlet, est effectué grâce à des soudures par ultrasons. Mais il est également possible de recourir en lieu et place du soudage à un assemblage par couture.

Afin d'assurer un déroulement parfait des bandes composant la bande multicouche, il est avantageusement prévu selon l'invention que la tension des bandes de gaze de coton hydrophile est obtenue par la traction exercée sur lesdites bandes par un dispositif d'entraînement et le freinage des bobines ou dérouleurs correspondant, tandis que la tension de la bande de non tissé est obtenue, après déroulement motorisé du dérouleur ou bobine correspondant, grâce à un dispositif de réglage automatique de la tension de ladite bande, disposé entre le dérouleur et le dispositif d'entraînement, et, grâce à l'entraînement par frottement de ladite bande de non tissé entre les bandes de gaze de coton hydrophile elles-mêmes entraînées par le dispositif d'entraînement. Il peut être prévu également un dispositif de réglage automatique de la tension de la bande multicouche, qui est intercalé entre, d'une part, la station de pose des rubans ou biais longitudinaux et / ou la station de soudage, et, d'autre part, la station de découpe des champs opératoires individuels.

Selon une autre caractéristique de l'invention, il est avantageusement prévu d'intégrer un fil radio-détectable dans au moins l'un des ourlets des champs opératoires. A cette fin, par exemple un fil en polyester imprégné de sulfate de baryum est posé le long d'au moins l'un des bords longitudinaux de la bande multicouche, préalablement ou simultanément à la pose des rubans ou biais et à leur solidarisation par une soudure par ultrasons ou par couture. Bien évidemment, il est également possible d'intégrer de manière analogue un fil radio-détectable dans au moins l'un des ourlets transversaux des champs opératoires.

En vue de maintenir les nappes composant la bande multicouche / les champs opératoires en un complexe unique et d'éviter qu'elles ne soient séparées les unes des autres avec le risque d'une détérioration du champ opératoire, il est avantageusement prévu d'appliquer, de préférence avant la pose des biais le long des bords longitudinaux et en tout cas avant la découpe des champs individuels, plusieurs soudures par ultrasons ou plusieurs coutures longitudinales réparties à intervalles réguliers sur toute la largeur de la bande multicouche / des champs opératoires.

La compresse ou le champ opératoire à usage unique fabriqué à l'aide du procédé conforme à l'invention est constitué de plusieurs couches, de préférence de trois couches, alternativement en gaze de coton hydrophile et en voile de non tissé hydrophile, les couches extérieures étant en gaze de coton hydrophile; ce champ opératoire présente des bords finis par un ruban ou biais en non tissé formant ourlet, solidarisé avec les nappes constitutives par soudure par ultrasons ou par des coutures, un fil radio-détectable étant intégré dans au moins l'un des quatre ourlets, et, il est pourvu de soudures par ultrasons ou de coutures longitudinales distantes les unes des autres, réparties sur toute la largeur du champs opératoire.

L'invention sera explicitée de façon purement indicative à l'aide des dessins annexés à titre d'exemples non limitatifs.

La figure 1A présente une coupe partielle d'un champ opératoire à trois couches, fabriqué selon le procédé conforme à l'invention.

La figure 1B présente une coupe partielle d'un champ opératoire à cinq couches, fabriqué selon le procédé conforme à l'invention.

La figure 2 est une vue partielle en perspective cavalière et en écorché d'un champ opératoire fabriqué selon le procédé conforme à l'invention.

La figure 3 présente une vue de côté schématique d'un plan de machine utilisée pour la mise en oeuvre du procédé de fabrication conforme à l'invention.

L'on trouvera in fine une nomenclature des différents repères utilisés au cours de la description.

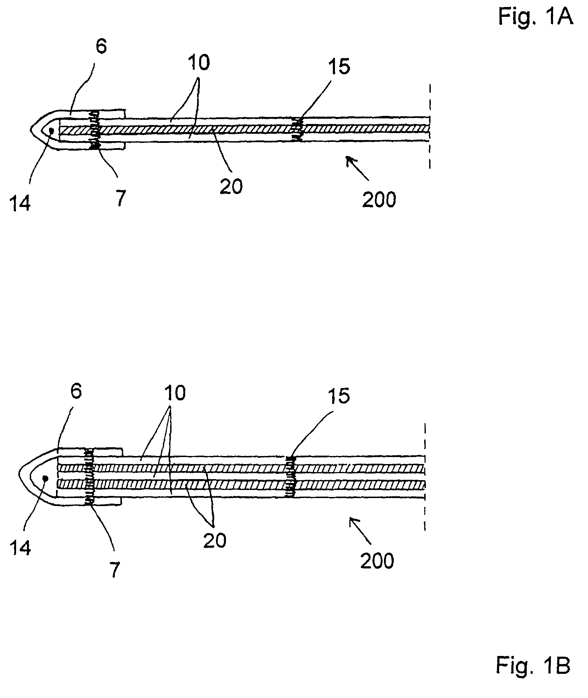

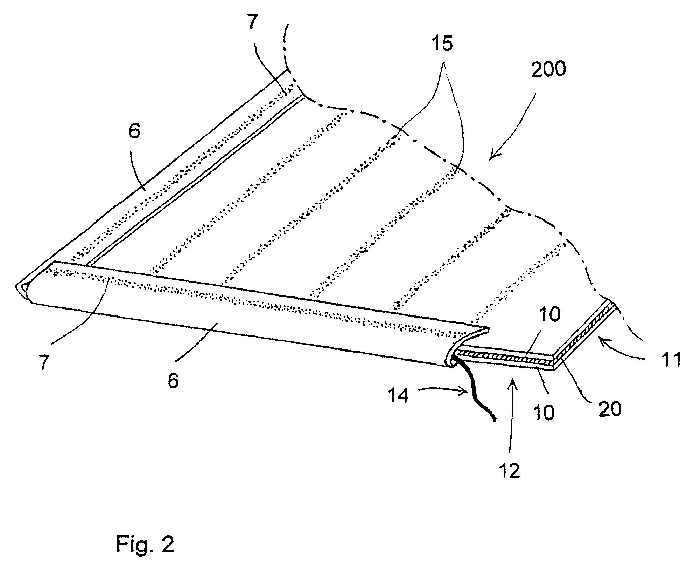

Les figures 1A et 1B présentent une coupe partielle de deux variantes de réalisation d'un champ opératoire à usage unique (200) conforme à et fabriqué selon le procédé de l'invention. La figure 2 présente une coupe partielle en perspective cavalière et en écorché du champ opératoire montré à la figure 1A.

Le champ opératoire (200) conforme aux figures 1 A et 2 se compose de deux nappes extérieures (10) en gaze de coton hydrophile entre lesquelles est disposée une nappe intérieure (20) en matériau non tissé. Dans le cadre de l'invention et comme montré à la figure 1B, le champ opératoire multicouche peut également présenter cinq ou davantage de nappes superposées, les nappes extérieures étant toujours en gaze de coton hydrophile. Selon l'usage en chirurgie, les nappes de gaze de coton hydrophile sont de préférence de couleur verte ou bleue afin d'éviter tout éblouissement.

La bande multicouche à partir de laquelle sont confectionnés les champs opératoires individuels (200) présente une largeur adaptée à l'usage envisagé, comprise généralement entre 40 cm et 100 cm. La gaze de coton hydrophile (10) présente un duitage variant en fonction de l'utilisation envisagée et du degré d'absorption recherché pour le champ opératoire, compris de préférence entre 13 fils / cm² et 20 fils / cm². Le poids de la gaze de coton varie généralement entre 30 g et 100 g / m².

La nappe intercalaire (20) est constitué d'un matériau de non tissé adapté au degré d'absorption recherché. Il peut s'agir avantageusement de viscose absorbante/polypropylène, polyester/viscose, polyester, polyamide.

L'association de ces deux matériaux utilisés respectivement pour les couches (10) et la couche (20) permet de cumuler les avantages inhérents à chacun d'eux, notamment la solidité et le « toucher tissu » de la gaze de coton et le grand pouvoir d'absorption du non tissé.

Les quatre bords des champs opératoires (200) conformes aux figures 1A, 1B et 2, sont finis par un ruban ou biais (6) en matériau non tissé, replié sur les nappes extérieures (10), formant ourlet, solidarisé par des soudures par ultrasons (7) traversant toutes les épaisseurs, à savoir les branches repliées du biais (6) et les nappes (10), (20). Les nappes du champ opératoire (200) sont pourvus de soudures (15) longitudinales par ultrasons, réparties sur toute sa largeur, distantes les unes des autres par exemple selon un pas de 5 cm à 8 cm, contribuant ainsi à solidariser les couches constitutives du champ opératoire et d'éviter qu'elles ne se séparent les unes des autres, risquant de détériorer le champ opératoire.

Selon l'invention, il est avantageusement prévu d'intégrer un fil radio-détectable (14) dans au moins l'un des quatre ourlets du champ opératoire (200). Il s'agit par exemple d'un fil de polyester imprégné de sulfate de baryum. Ce fil radio-détectable, amené à partir d'une bobine ou d'un dérouleur correspondant, est avantageusement posé préalablement ou simultanément à la pose du ruban ou biais (6) sur les bords longitudinaux (11) de la bande multicouche (100). Il est possible d'intégrer en outre de manière analogue un fil radio-détectable dans l'ourlet du second bord longitudinal (11) et/ou dans au moins l'un des ourlets des bords transversaux (12).

Le ruban ou biais (6) en matériau non tissé, replié sur les faces extérieures du champ opératoire, borde de près les chants longitudinaux (11) et transversaux (12) de celui-ci, mais un débordement jusqu'à environ 5 mm du biais (6) par rapport aux chants du champ opératoire est admissible.

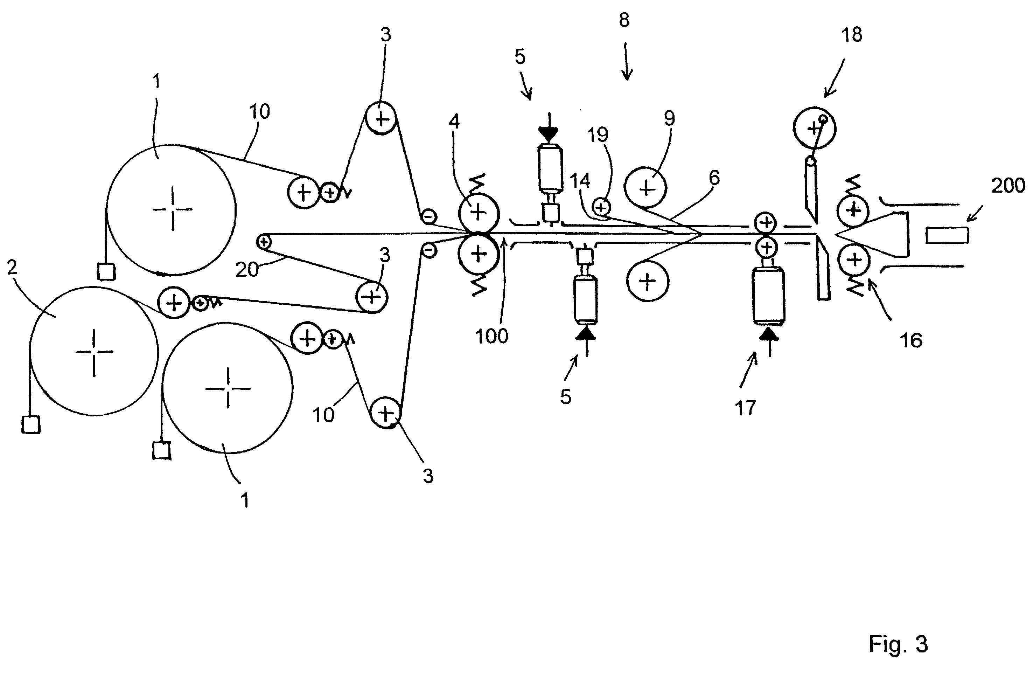

Ce champ opératoire à usage unique est fabriqué selon le procédé en continu proposé par l'invention. Le déroulement de ce procédé, illustré à titre d'exemple par le plan de machine schématique montré à la figure 3, comprend les étapes suivantes:
- confection d'une bande ou nappe multicouche (100) unique continue par déroulement simultané de bandes ou nappes de gaze de coton hydrophile (10) et de voile de non tissé (20) à partir de dérouleurs (1), (2) auxquels peut être associé au moins un dispositif (3) de réglage automatique de la tension des bandes, lesdites bandes (10), (20) passant ensemble par un dispositif d'entraînement motorisé (4) avant d'atteindre les stations de confection (5), (8), (17), (18) des champs opératoires individuels (200),
- pose, par déroulement à partir de dérouleurs (9), de rubans ou biais (6) continus en non tissé sur les bords longitudinaux (11) de la bande multicouche (100), lesdits biais étant repliés autour des bords longitudinaux de la bande multicouche sur les nappes extérieures en gaze de coton hydrophile (10) pour former ourlet, cette opération étant effectuée à une station de pose (8) des rubans ou biais,
- assemblage, à une station de soudage (17), des rubans ou biais (6) repliés autour des bords longitudinaux (11) de la bande multicouche (100) par des soudures par ultrasons (7) traversant les branches repliées des biais (8) et toutes les couches de la bande multicouche (100),
- découpe transversale à longueur des champs opératoires individuels (200) à une station de découpe (18) et éventuellement aspiration des poussières par un dispositif d'aspiration,
- reprise en perpendiculaire du champ opératoire découpé (200) par un dispositif d'entraînement (16),
- pose de rubans ou biais (6) formant ourlet autour des bords transversaux (12) du champ opératoire découpé, lesdits rubans ou biais repliés étant solidarisés par des soudures par ultrasons (7).

Toujours selon l'invention, il peut être avantageusement prévu d'appliquer, de préférence préalablement à la découpe des champs opératoires individuels (200) plusieurs soudures par ultrasons (15) longitudinales, réparties à intervalles réguliers sur toute la largeur de la bande multicouche, ces soudures étant effectuées de préférence à une station de soudage (5) disposée en amont de la station de pose (8) des rubans ou biais (6).

Selon un mode de réalisation préférentiel, il est prévu de poser un fil radio-détectable (14), par exemple un fil en polyester imprégné de sulfate de baryum, le long d'au moins l'un des bords longitudinaux de la bande multicouche (100). Ce fil radio-détectable (14) est avantageusement posé le long d'au moins l'un des bords longitudinaux de la bande multicouche (100). Le fil radio-détectable (14) est posé de préférence préalablement ou simultanément à la pose du ruban ou biais (6). A cet effet, la bobine ou le dérouleur (19) de fil radio-détectable (14) est avantageusement disposé immédiatement en amont du dérouleur (9) de biais (6) de façon à intégrer le fil radio-détectable (14) dans l'ourlet correspondant. Le bon déroulement du fil radio-détectable (14) et des rubans ou biais (6) peut être assurée grâce à un dispositif de réglage automatique de la tension.

Afin d'assurer un déroulement parfait des bandes (10, 20) composant la bande multicouche (100) à partir de laquelle sont confectionnés les champs opératoires individuels, il est avantageusement prévu selon l'invention que la tension des bandes de gaze de coton hydrophile est obtenue par la traction exercée sur lesdites bandes par un dispositif d'entraînement (4) et le freinage des bobines ou dérouleurs correspondants (1), tandis que la tension de la bande de non tissé (20) est obtenue, après déroulement motorisé du dérouleur ou bobine correspondant (2), grâce à un dispositif (3) de réglage automatique de la tension de ladite bande (100), disposé entre le dérouleur (2) et le dispositif d'entraînement (4), et, grâce à l'entraînement par frottement de ladite bande de non tissé (2) entre les bandes de gaze de coton hydrophile (10) elles-mêmes entraînées par le dispositif d'entraînement (4). Il peut être prévu également un dispositif de réglage automatique de la tension de la bande multicouche, intercalé par exemple entre, d'une part, la station de pose (8) des rubans ou biais longitudinaux et / ou la station de soudage (17), et, d'autre part, la station de découpe (18) des champs opératoires individuels (200).

Selon un mode de réalisation préférentiel de l'invention, l'assemblage des nappes (10), (20) composant la bande multicouche (100) et la fixation des rubans ou biais (6) repliés autour des quatre côtés des champs opératoires pour former ourlet, est effectué grâce à des soudures par ultrasons (7). Mais il est également possible de recourir en lieu et place du soudage à un assemblage par couture. Ainsi, les soudures par ultrasons longitudinales (15) réparties sur toutes la largeur du champs opératoire ainsi que les soudures par ultrasons (7) d'assemblages des biais (6) bordant les chants du produit fini seront remplacées par des coutures effectués à des stations de couture de manière analogue et aux étapes correspondantes du procédé de fabrication explicité précédemment.

### Nomenclature

- 1: bobine ou dérouleur de la bande de gaze de coton hydrophile
- 2: bobine ou dérouleur de la bande de voile de non tissé
- 3: dispositif de réglage automatique de la tension
- 4: dispositif d'entraînement
- 5: station de soudage des soudures longitudinales (15)
- 6: ruban ou biais en matériau non tissé
- 7: soudures par ultrasons d'assemblage des biais (6)
- 8: station de pose des biais (6)
- 9: bobine ou dérouleur du biais (6)
- 10: bande ou nappe de gaze de coton hydrophile
- 11: bords longitudinaux de la bande multicouche (100)
- 12: bords transversaux de la bande multicouche (100)
- 14: fil radio-détectable
- 15: soudures longitudinales par ultrasons
- 16: dispositif d'entraînement
- 17: station de soudage
- 18: station de découpe des champs individuels
- 19: bobine ou dérouleur du fil radio-détectable (14)
- 20: bande ou nappe en matériau non tissé
- 100: bande multicouche continue
- 200: compresse ou champ opératoire individualisé

## Revendications

1. Procédé de fabrication en continu de compresses ou champs opératoires à usage unique, constitués de plusieurs nappes superposées alternativement en gaze de coton hydrophile (10) et en voile de non tissé (20) hydrophile, les nappes extérieures (10) étant en gaze de coton hydrophile, **caractérisé par** les étapes suivantes :
• confection d'une bande ou nappe multicouche (100) unique continue par déroulement simultané de bandes ou nappes de gaze de coton hydrophile (10) et de voile de non tissé (20) à partir de bobines ou dérouleurs (1), (2) auxquels peut être associé au moins un dispositif (3) de réglage automatique de la tension des bandes, lesdites bandes (10), (20) passant ensemble par un dispositif d'entraînement (4) motorisé avant d'atteindre les stations de confection (5), (8), (17), (18), des champs opératoires individuels,
• pose par déroulement à partir de dérouleurs (9) de rubans ou biais (6) continus en non tissé sur les bords longitudinaux (11) de la bande multicouche (100), lesdits biais (6) étant repliés autours des bords longitudinaux de la bande multicouche sur les nappes extérieures (10) pour former ourlet,
• assemblage, à une station de soudage (17), des rubans ou biais (6) repliés autour des bords longitudinaux (11) de la bande multicouche (100) par des soudures (7) par ultrasons traversant les branches repliées du biais (6) et toutes les couches de la bande multicouche (100),
• découpe transversale à longueur des champs opératoires individuels (200) à une station de découpe (18) et éventuellement aspiration des poussières par un dispositif d'aspiration,
• reprise en perpendiculaire du champ opératoire découpé (200) par un dispositif d'entraînement (16),
• pose de rubans ou biais (6) formant ourlet autour des bords transversaux (12) du champ opératoire individualisé (200) et solidarisation desdits rubans ou biais repliés par des soudures par ultrasons (7).

2. Procédé selon la revendication 1 **caractérisé par** l'application, préalable à la découpe des champs individuels (200) de plusieurs soudures par ultrasons (15) longitudinales réparties à intervalles réguliers sur toute la largeur de la bande multicouche (100).

3. Procédé de fabrication en continu de compresses ou champs opératoires à usage unique, constitués de plusieurs nappes superposées alternativement en gaze de coton hydrophile (10) et en voile de non tissé (20) hydrophile, les nappes extérieures (10) étant en gaze de coton hydrophile, **caractérisé par** les étapes suivantes :
• confection d'une bande ou nappe multicouche (100) unique continue par déroulement simultané de bandes ou nappes de gaze de coton hydrophile (10) et de voile de non tissé (20) à partir de bobines ou dérouleurs (1), (2) auxquels peut être associé au moins un dispositif (3) de réglage automatique de la tension des bandes, lesdites bandes (10), (20) passant ensemble par un dispositif d'entraînement (4) motorisé avant d'atteindre les stations de confection (8), (18), des champs opératoires individuels,
• pose par déroulement à partir de dérouleurs (9) de rubans ou biais (6) continus en non tissé sur les bords longitudinaux (11) de la bande multicouche (100), lesdits biais (6) étant repliés autours des bords longitudinaux de la bande multicouche sur les nappes extérieures (10) pour former ourlet,
• assemblage, à une station de couture, des rubans ou biais (6) repliés autour des bords longitudinaux (11) de la bande multicouche (100) par des coutures traversant les branches repliées des biais (6) et toutes les couches de la bande multicouche (100),
• découpe transversale à longueur des champs opératoires individuels (200) à une station de découpe (18) et éventuellement aspiration des poussières par un dispositif d'aspiration,
• reprise en perpendiculaire du champ opératoire découpé (200) par un dispositif d'entraînement (16),
• pose de rubans ou biais (6) formant ourlet autour des bords transversaux (12) du champ opératoire individualisé (200) et solidarisation desdits rubans ou biais repliés par des coutures.

4. Procédé selon la revendication 3 **caractérisé par** l'application, préalable à la découpe des champs individuels (200) de plusieurs coutures longitudinales réparties à intervalles réguliers sur toute la largeur de la bande multicouche (100).

5. Procédé selon les revendications 1 et 2 ou 3 et 4 **caractérisé en ce que** la tension des bandes de gaze de coton hydrophile (10) est obtenue par la traction exercée sur lesdites bandes par un dispositif d'entraînement (4) et le freinage des bobines ou dérouleurs (1), et **en ce que** la tension de la bande de non tissé (20) est obtenue après déroulement motorisé du dérouleur ou bobine (2) grâce à un dispositif de réglage automatique de la tension (3) de ladite bande, disposé entre le dérouleur (2) et le dispositif d'entraînement (4), et, grâce à l'entraînement par frottement de la bande (20) entre les bandes de gaze de coton hydrophile (10) entraînées par le dispositif d'entraînement (4).

6. Procédé selon l'une quelconque des revendications précédentes **caractérisé par** l'utilisation d'un dispositif de réglage automatique de la tension de la bande multicouche (100), intercalé entre la station de pose (8) des rubans ou biais longitudinaux (6) et / ou la station de soudage (17), et, la station de découpe (18) des champs opératoires individuels (200).

7. Procédé selon l'une quelconque des revendications précédentes **caractérisé par** la pose d'un fil (14) radio-détectable le long d'au moins l'un des bords longitudinaux (11) de la bande multicouche (100) préalable ou simultanée à la pose des rubans ou biais (6) formant ourlet afin d'intégrer ledit fil radio-détectable (14) dans le ou les ourlets.

8. Procédé selon l'une quelconque des revendications précédentes **caractérisé par** l'intégration d'un fil radio-détectable (14) dans au moins l'un des ourlets autour des bords transversaux (12) du champ opératoire individuel (200).

9. Compresse ou champ opératoire à usage unique, constitué de plusieurs couches superposées alternativement en gaze de coton hydrophile (10) et en voile de non tissé (20) hydrophile, les couches extérieures (10) étant en gaze de coton hydrophile, qui est fabriqué à l'aide du procédé conforme aux revendications précédentes **caractérisé en ce que** il est pourvu de bords finis par un ruban ou biais (6) en non tissé replié pour former ourlet, solidarisé par des soudures par ultrasons (7) / par des coutures, un fil (14) radio-détectable étant intégré dans au moins l'un des quatre ourlets.

10. Compresse ou champ opératoire à usage unique selon la revendication 9 **caractérisé en ce que** il est pourvu de soudures par ultrasons (15) / coutures longitudinales réparties sur toute sa largeur, contribuant à solidariser les couches constitutives.

## Patentansprüche

1. Verfahren zur kontinuierlichen Herstellung einer Kompresse oder eines chirurgischen Abdecktuchs für einmalige Verwendung, bestehend aus mehreren übereinander angeordneten Schichten, wechselweise aus saugfähiger Baumwollgaze (10) und aus saugfähigem Vliesstoff (20), wobei die äußeren Schichten (10) aus saugfähiger Baumwollgaze sind, **gekennzeichnet durch** die folgenden Verfahrensschritte :
- Herstellen eines einzigen fortlaufenden, vielschichtigen Bandes (100) **durch** gleichzeitiges Abwickeln von Bändern aus saugfähiger Baumwollgaze (10) und Bändern aus Vliesstoff (20) ausgehend von Spulen (1), (2), welche mit mindestens einer Vorrichtung (3) zur automatischen Spannungsregelung der Bänder zusammenwirken, wobei die Bänder (10), (20) zusammen **durch** eine motorisierte Antriebsvorrichtung (4) hindurchlaufen, bevor sie Stationen (5), (8), (17), (18) zur Anfertigung der einzelnen chirurgischen Abdecktücher erreichen,
- Anbringen auf die Längskanten (11) des vielschichtigen Bandes (100) von fortlaufenden, von Spulen (9) abgewickelten Streifen oder Schrägstreifen (6) aus Vliesstoff, welche um die Längskanten des vielschichtigen Bandes (100) herumgefaltet werden und zur Saumbildung auf die äußeren Schichten (10) aufliegen,
- Befestigung der um die Längskanten (11) des vielschichtigen Bandes (100) herumgefalteten Streifen oder Schrägstreifen (6) **durch** Ultraschallschweißen an einer Schweißstation, wobei die Schweißnähte alle Schichten des vielschichtigen Bandes (100) durchqueren,
- Erfassen in Querrichtung des abgeschnittenen chirurgischen Abdecktuchs (200) **durch** eine Mitnahmevorrichtung (16),
- Anbringen von saumbildenden Streifen oder Schrägstreifen (6) um die Querkanten (12) des einzelnen chirurgischen Abdecktuchs (200) herum und Befestigen der Streifen oder Schrägstreifen (6) **durch** Ultraschallschweißen (7).

2. Verfahren nach Anspruch 1, **dadurch gekennzeichnet, dass** vor dem Abschneiden der einzelnen chirurgischen Abdecktücher (200) mehrere in regelmäßigen Abständen über die gesamte Breite des vielschichtigen Bandes (100) verteilte Längsschweißnähte (15) durch Ultraschallschweißen angebracht werden.

3. Verfahren zur kontinuierlichen Herstellung einer Kompresse oder eines chirurgischen Abdecktuchs für einmalige Verwendung, bestehend aus mehreren übereinander angeordneten Schichten, wechselweise aus saugfähiger Baumwollgaze (10) und aus saugfähigem Vliesstoff (20), wobei die äußeren Schichten (10) aus saugfähiger Baumwollgaze sind, **gekennzeichnet durch** die folgenden Verfahrensschritte :
- Herstellen eines einzigen fortlaufenden, vielschichtigen Bandes (100) **durch** gleichzeitiges Abwickeln von Bändern aus saugfähiger Baumwollgaze (10) und Bändern aus Vliesstoff (20) ausgehend von Spulen (1), (2), welche mit mindestens einer Vorrichtung (3) zur automatischen Spannungsregelung der Bänder zusammenwirken, wobei die Bänder (10), (20) zusammen **durch** eine motorisierte Antriebsvorrichtung (4) hindurchlaufen, bevor sie Stationen (5), (8), (17), (18) zur Anfertigung der einzelnen chirurgischen Abdecktücher erreichen,
- Anbringen auf die Längskanten (11) des vielschichtigen Bandes (100) von fortlaufenden, von Spulen (9) abgewickelten Streifen oder Schrägstreifen (6) aus Vliesstoff, welche um die Längskanten des vielschichtigen Bandes (100) herumgefaltet werden und zur Saumbildung auf die äußeren Schichten (10) aufliegen,
- Befestigung der um die Längskanten (11) des vielschichtigen Bandes (100) herumgefalteten Streifen oder Schrägstreifen (6) **durch** Nähen an einer Nähstation, wobei die Nähte alle Schichten des vielschichtigen Bandes (100) durchqueren,
- Erfassen in Querrichtung des abgeschnittenen chirurgischen Abdecktuchs (200) **durch** eine Mitnahmevorrichtung (16),
- Anbringen von saumbildenden Streifen oder Schrägstreifen (6) um die Querkanten (12) des einzelnen chirurgischen Abdecktuchs (200) herum und Befestigen der Streifen oder Schrägstreifen (6) **durch** Anbringen von Nähten.

4. Verfahren nach Anspruch 3, **dadurch gekennzeichnet, dass** vor dem Abschneiden der einzelnen chirurgischen Abdecktücher (200) mehrere in regelmäßigen Abständen über die gesamte Breite des vielschichtigen Bandes (100) verteilte Nähte (15) durch Nähen angebracht werden.

5. Verfahren nach den Ansprüchen 1 und 2 oder 3 und 4, **dadurch gekennzeichnet, dass** die Spannung der Bänder (10) aus saugfähiger Baumwollgaze durch den über die Antriebsvorrichtung (4) auf die Bänder (10) ausgeübten Zug und durch das Abbremsen der Spulen (1) bewirkt wird und dass die Spannung des Bandes (20) aus Vliesstoff nach dem motorisierten Abwickeln von der Spule (2) einerseits über eine zwischen der Spule (2) und der Antriebsvorrichtung (4) angeordneten Vorrichtung (3) zur automatischen Spannungsregelung und andererseits über die Mitnahme durch Haftreibung des zwischen den über die Antriebsvorrichtung (4) mitgenommenen Bänder (10) aus saugfähiger Baumwollgaze angeordneten Bandes (20) aus Vliesstoff bewirkt wird.

6. Verfahren nach einem der vorangehenden Ansprüche, **gekennzeichnet durch** den Einsatz einer Vorrichtung zur automatischen Spannungsregelung des vielschichtigen Bandes (100), die zwischen einerseits der Station (8) zum Anbringen der Streifen oder Schrägstreifen (6) und/oder der Schweißstation (17) und andererseits der Station (18) zum Abschneiden der einzelnen chirurgischen Abdecktücher angeordnet ist.

7. Verfahren nach einem der vorangehenden Ansprüche, **dadurch gekennzeichnet, dass** entlang wenigstens einer der Längskanten (11) des vielschichtigen Bandes (100) ein mittels Durchstrahlung nachweisbarer Faden (14) angebracht wird, vor oder gleichzeitig zur Anbringung der saumbildenden, den mittels Durchstrahlung nachweisbaren Faden (14) einschließenden Streifen oder Schrägstreifen (6).

8. Verfahren nach einem der vorangehenden Ansprüche, **dadurch gekennzeichnet, dass** ein mittels Durchstrahlung nachweisbarer Faden (14) in wenigstens einem der die Querkanten (12) der einzelnen Abdecktücher (200) abschließenden Säume eingefügt ist.

9. Kompresse oder chirurgisches Abdecktuch für einmalige Verwendung, bestehend aus mehreren übereinander angeordneten Schichten, wechselweise aus saugfähiger Baumwollgaze (10) und aus saugfähigem Vliesstoff (20), wobei die äußeren Schichten (10) aus saugfähiger Baumwollgaze sind und wobei die Kompresse oder das chirurgische Abdecktuch mittels des Verfahrens gemäß den vorangehenden Ansprüchen hergestellt ist, **dadurch gekennzeichnet, dass** seine Kanten durch einen um diese herumgefalteten, saumbildenden Streifen oder Schrägstreifen (6) aus Vliesstoff fertiggearbeitet sind, wobei die Streifen oder Schrägstreifen (6) mittels Ultraschallschweißen oder Nähen befestigt sind und ein mittels Durchstrahlung nachweisbarer Faden (14) in wenigstens einen der vier Säume eingefügt ist.

10. Kompresse oder chirurgisches Abdecktuch nach Anspruch 9, **gekennzeichnet durch** mittels Ultraschallschweißen oder Nähen angebrachte, über die gesamte Breite des chirurgischen Abdecktuchs verteilte, die Verbindung der verschiedenen Schichten bezweckende Längsnähte.

## Claims

1. A method of continuously manufacturing compresses or surgical drapes for single use, constituted by a plurality of superposed alternating sheets of hydrophilic cotton gauze (10) and of non woven hydrophilic web (20), the outer sheets (10) being of hydrophilic cotton gauze, the method being **characterized by** the following steps :
- a single continuous multiplayer strip or sheet (100) is made by simultaneously paying out strips or sheets of hydrophilic cotton gauze (10) and of non-woven web (20) from payout spools or reels (1, 2), which may be associated with at least one device (3) for automatically adjusting the tension of the strips, said strips (10, 20) passing together via a motorized drive device (4) prior to reaching stations (5, 8, 17, 18) for making up individual surgical drapes;
- continuous non-woven ribbons or bias bindings (6) are put into place on the longitudinal edges (11) of the multiplayer strip (100) by being paid out from spools (9), said bias bindings (6) being folded around the longitudinal edges of the multiplayer strip over the outer sheets (10) to form hems;
- the ribbons or bias bindings (6) folded around the longitudinal edges (11) of the multiplayer strip (100) are assembled in a bonding station (17) by means of ultrasonic bonds (7) passing through the folded-over branches of the bias binding (6) and through all of the layers of the multiplayer strip (100);
- individual surgical drapes (200) are cut transversely to length in a cutting station (18) and optionally dust is sucked up by means of a suction device;
- the cut off surgical drape (200) is taken up perpendicularly by a drive device (16); and
- hem-forming ribbons or bias bindings (6) are placed around the transverse edges (12) of the individualized surgical drapes (200) and are secured to the latter by ultrasonic bonding (7).

2. A method according to claim 1, **characterized by** applying a plurality of longitudinal lines of ultrasonic bonding (15) that are distributed at regular intervals over the entire width of the multiplayer strip (100) prior to the strip being cut up into individual surgical drapes (200).

3. A method of continuously manufacturing compresses or surgical drapes for single use, constituted by a plurality of superposed alternating sheets of hydrophilic cotton gauze (10) and of non woven hydrophilic web (20), the outer sheets (10) being of hydrophilic cotton gauze, the method being **characterized by** the following steps :
- a single continuous multiplayer strip or sheet (100) is made by simultaneously paying out strips or sheets of hydrophilic cotton gauze (10) and of non-woven web (20) from payout spools or reels (1, 2), which may be associated with at least one device (3) for automatically adjusting the tension of the strips, said strips (10, 20) passing together via a motorized drive device (4) prior to reaching stations (5, 8, 17, 18) for making up individual surgical drapes;
- continuous non-woven ribbons or bias bindings (6) are put into place on the longitudinal edges (11) of the multiplayer strip (100) by being paid out from spools (9), said bias bindings (6) being folded around the longitudinal edges of the multiplayer strip over the outer sheets (10) to form hems;
- the ribbons or bias bindings (6) folded around the longitudinal edges (11) of the multiplayer strip (100) are assembled in a stitching station (17) by means of stitching passing through the folded-over branches of the bias binding (6) and through all of the layers of the multiplayer strip (100);
- individual surgical drapes (200) are cut transversely to length in a cutting station (18) and optionally dust is sucked up by means of a suction device;
- the cut off surgical drape (200) is taken up perpendicularly by a drive device (16); and
- hem-forming ribbons or bias bindings (6) are placed around the transverse edges (12) of the individualized surgical drapes (200) and are secured to the latter by stitching.

4. A method according to claim 3, **characterized by** applying a plurality of longitudinal lines of stitching that are distributed at regular intervals over the entire width of the multiplayer strip (100) prior to the strip being cut up into individual surgical drapes (200).

5. A method according to claims 1 and 2 or 3 and 4, **characterized in that** the tension of the strips of hydrophilic cotton gauze (10) is obtained by traction exerted on said strips by a drive device (4) and by braking the payout spools or reels (1), and **in that** the tension of the non-woven strip (20) is obtained after motor-driven payout from the spool or reel (2) by means of a device (3) for automatically adjusting the tension of said strip, which device (3) is placed between the payout spool (2) and the drive device (4), and because of the strip (20) being driven by friction between the strips of the hydrophilic cotton gauze (10), themselves driven by the drive device (4).

6. A method according to any precedent claim, **characterized by** the use of a device for automatically adjusting the tension of the multiplayer strip (100), which device is interposed between the station for putting the longitudinal ribbons or bias bindings (6) into place and/or the bonding station (17) and the station (18) for cutting up the individual surgical drapes (200).

7. A method according to any precedent claim, **characterized by** an X-ray detectable thread (14) being put into place along at least one of the longitudinal edges (11) of the multiplayer strip (100) before or simultaneously with the hem-forming ribbons or bias bindings (6) being put into place, so as to integrate said X-ray detectable thread (14) in the hem(s).

8. A method according to any preceding claim, **characterized by** an X-ray detectable thread (14) being integrated in at least one of the hems around the transverse edges (12) of the individual surgical drapes (200).

9. A compress or surgical drape for single use constituted by a plurality of superposed alternating sheets of hydrophilic cotton gauze (10) and of non-woven hydrophilic web (20), the outer sheets (10) being of hydrophilic cotton gauze, the said compress being manufactured by using the method according to any precedent claim, **characterized in that** it is provided with edges finished by means of respective non-woven ribbons or bias bindings (6) that form hems, secured by ultrasonic bonding (7) or by stitching, an X-ray detectable thread (14) being integrated in at least one of the four hems.

10. A compress or surgical drape for single use according to claim 9 **characterized in that** it is provided with longitudinal lines of ultrasonic bonding (15) or of stitching distributed over its entire width, contributing to securing the component layers to one other.
